# EUROPEAN PATENT APPLICATION

(11) **EP 4 435 799 A1**
(43) Date of publication of application: **25.09.2024**
(21) Application number: 23163213.4
(22) Date of filing: 21.03.2023
(51) Int. Cl.: G16H 50/20, G16H 50/30

(54) **SYSTEM AND METHOD FOR PROVIDING PREDICTION FOR PHYSIOLOGICAL PARAMETER OF PATIENT**

(71) Applicant: ETH Zürich, 8092 Zürich (CH)
(72) Inventor: DUBATOVKA, Alina, 8051 Zürich (CH)
(74) Representative: DeltaPatents B.V.

(57) **Abstract**

Systems and methods are provided to provide a prediction for a physiological parameter of a patient to a clinical decision support tool. For that purpose, a real-time or near real-time data stream is accessed of measured values of a physiological parameter of a patient. A trained machine learning model is used to generate, using a time-series of measured values from the real-time or near real-time stream as input, a time-series of predicted values as output. It is determined whether predicted values fall within a physiological range associated with the physiological parameter, thereby obtaining a time-series of classifications, wherein a respective classification is indicative of whether a predicted value falls inside or outside of the physiological range. The time-series of classifications provided to the clinical decision support tool, for example to visualize the time-series of classifications in a graphical representation of a patient's medical condition.

## Description

### FIELD OF THE INVENTION

The invention relates to a prediction system and computer-implemented method for providing a prediction for a physiological parameter of a patient and for outputting the prediction to a clinical decision support tool. The invention further relates to a patient monitor comprising the prediction system. The invention relates to a training system and computer-implemented method for training a machine learning model to predict values of a physiological parameter of a patient. The invention further relates to a computer-readable medium comprising instructions for a computer program, the computer program comprising instructions to cause a processor system to perform the computer-implemented method.

### BACKGROUND OF THE INVENTION

It is known to monitor physiological parameters of a subject at regular and consecutive time instances. For example, the heartrate of a runner may be monitored to serve as a performance metric during a run. If the monitoring is in the context of medical care, the subject may also be referred to as patient and such monitoring as patient monitoring. In both cases, the measured values of the physiological parameters may be visualized, for example for the user or patient to see (e.g., in self-care scenarios) or for medical personnel to see. Typically, in patient monitoring, the measured values may be compared to static or dynamic thresholds to detect abnormalities in the physical parameters, and if such abnormalities are detected, an alarm may be triggered, e.g., to attract the attention of medical personnel. For example, on an intensive care unit (ICU), a patient's vital signs (heart rate (HR), respiration rate (RR), core body temperature (CBT), oxygen saturation (SpO2) and blood pressure (BP) may be closely monitored and compared to a normal or acceptable physiological range, and the alarm may be triggered if the value of a physiological parameter exceeds or falls below the range. In general, physiological parameters may include, but are not limited to, vital signs such as heart rate, respiration rate, and blood pressure, advanced hemodynamic parameters such as cardiac output, stroke volume, and systemic vascular resistance, hematological parameters, such as red and white blood cell counts and hemoglobin concentration, and analyte parameters, such as glucose or lactate, concentrations in whole blood, plasma, or serum.

It is known in patient monitoring to show a trend view of the measured values of the physiological parameters on a display. Such a trend view may provide a longitudinal visualization of the measurement data by setting out the measured values of the physiological parameters at the consecutive time instances against a common timeline. Typically, in such a trend view, the time is presented on the x-axis while the measured values of the physiological parameters are presented on the y-axis, typically in the form of graphs which are vertically separated from each other along the y-axis but horizontally aligned with respect to the time on the x-axis. Based on the trend view, medical personnel may spot trends in the measured physiological parameters, such as a deterioration in the medical condition of the patient, and intervene in the patient's treatment, if necessary.

However, it may require sufficient time to perceive and understand the information presented in a trend view. Such time is not always available, for example in an emergency situation or when medical personnel have to care for a large number of patients.

It is known to equip a patient monitor with a user interface that enables a user, such as medical personnel, to better perceive and understand the information presented by the patient monitor. For example, US 10702214B2 describes a system and method for visualizing a patient's medical condition, wherein a graphical representation of the patient comprising a body having at least a torso and a head, as well as particularly two legs and two arms, is displayed using a display device, wherein said displayed graphical representation comprises at least one region which is allocated to at least one or several provided (e.g. measured and/or determined) patient monitoring quantities, and wherein the appearance of the at least one region is altered in real-time when the at least one patient monitoring quantity to which said at least one region is allocated changes.

The graphical representation of the patient as described in US 10702214B2 addresses at least some of the disadvantages of a trend view. However, the graphical representation concerns a patient's current state. Medical personnel are frequently also concerned with the patient's future state and may attempt to, based on the presented information, mentally identify trends to try to identify whether or not physiological parameters of the patient remain in a normal or acceptable physiological range. Disadvantageously, such mental exercises may also be time-consuming and error-prone. It would therefore be desirable to enable the graphical representation of US 10702214B2, as well as other clinical decision support tools, to also concern themselves with a patient's future state without requiring a user to mentally identify trends on the basis of the presented information.

### SUMMARY OF THE INVENTION

In a first aspect of the invention, a prediction system is provided for providing a prediction for a physiological parameter of a patient and for outputting the prediction to a clinical decision support tool. The prediction system comprises:
an input interface configured to access a real-time or near real-time data stream of measured values of a physiological parameter of the patient;
a data storage comprising data defining machine learning model, wherein the machine learning model is configured to predict, using a first time-series of values of the physiological parameter as input, a second time-series of values of the physiological parameter, wherein the second time-series represents a numerical prediction of the values of the physiological parameter ahead in time of the first time series;
an output interface to the clinical decision support tool;
a processor subsystem configured to:
   - use the machine learning model to generate, using a time-series of measured values from the real-time or near real-time stream as input, a time-series of predicted values as output;
   - determine for the time-series of predicted values whether predicted values fall within a physiological range associated with the physiological parameter, thereby obtaining a time-series of classifications, wherein a respective classification is indicative of whether a predicted value falls inside or outside of the physiological range; and
   - provide the time-series of classifications to the clinical decision support tool.

In a further aspect of the invention, a computer-implemented method is provided for providing a prediction for a physiological parameter of a patient and for providing the prediction to a clinical decision support tool. The method comprises:
accessing a real-time or near real-time data stream of measured values of a physiological parameter of the patient;
accessing data defining machine learning model, wherein the machine learning model is configured to predict, using a first time-series of values of the physiological parameter as input, a second time-series of values of the physiological parameter, wherein the second time-series represents a numerical prediction of the values of the physiological parameter ahead in time of the first time series;
using the machine learning model to generate, using a time-series of measured values from the real-time or near real-time stream as input, a time-series of predicted values as output;
determining for the time-series of predicted values whether predicted values fall within a physiological range associated with the physiological parameter, thereby obtaining a time-series of classifications, wherein a respective classification is indicative of whether a predicted value falls inside or outside of the physiological range; and
providing the time-series of classifications to the clinical decision support tool.

In a further aspect of the invention, a training system is provided for training a machine learning model to predict future values of a physiological parameter of a patient based on past measured values of the physiological parameter. The training system comprises:
an input interface configured to access training data in form of recordings of real-time or near real-time data streams of measured values of a physiological parameter of one or more patients;
a processor subsystem configured to iteratively train the machine learning model on the training data to obtain a machine learning model by, in a training iteration:
   - selecting a first time-series of values of the physiological parameter from the training data;
   - selecting a second time-series of values of the physiological parameter from the training data, wherein the second time series is part of a same data stream as the first time-series of values of the physiological parameter and is ahead in time of the first time series;
   - training the machine learning model to predict the second time-series of values of the physiological parameter from the first time-series of values of the physiological parameter as input.

In a further aspect of the invention, a computer-implemented method is provided for training a machine learning model to predict future values of a physiological parameter of a patient based on past measured values of the physiological parameter. The method comprises:
accessing training data in form of recordings of real-time or near real-time data streams of measured values of a physiological parameter of one or more patients;
iteratively training the machine learning model on the training data to obtain a machine learning model by, in a training iteration:
   - selecting a first time-series of values of the physiological parameter from the training data;
   - selecting a second time-series of values of the physiological parameter from the training data, wherein the second time series is part of a same data stream as the first time-series of values of the physiological parameter and is ahead in time of the first time series;
   - training the machine learning model to predict the second time-series of values of the physiological parameter from the first time-series of values of the physiological parameter as input.

In a further aspect of the invention, a transitory or non-transitory computer-readable medium is provided, the computer-readable medium comprising data representing a computer program comprising instructions for causing a processor system to perform a computer-implemented method as described in this specification.

The above measures relate to patient monitoring, which may typically involve monitoring the condition of a patient in a clinical setting, e.g., in a care center, but which may also include non-clinical settings, e.g., in a self-care setting, as well as non-medical settings, e.g., when monitoring the performance of a user during exercise. In such patient monitoring, physiological parameters of the patient may be measured at consecutive time instances, for example at regular intervals, e.g., every 100ms, 1 sec, 1 minute, 10 minutes, etc., or at irregular intervals, e.g., when changes in the physiological parameters are expected to occur. For such measurements, various known types of sensors and/or sensing devices may be used, including but not limited to the types described elsewhere in this specification.

The above measures involve providing a prediction system which has real-time or near real-time access to the measured values of physiological parameters of a patient. For example, the prediction system may be connected to a patient monitor or may in some embodiments be a subsystem of a patient monitor. Another example is that the prediction system may be a workstation or mobile device which has remote access to the measured values of the physiological parameters measured by such a patient monitor. In yet another example, the prediction system may be directly connected to one or more sensors which are arranged for measuring one or more physiological parameters of the patient.

The above measures further involve providing a machine learning model which is configured, by training, to predict, using a first time-series of values of the physiological parameter as input, a second time-series of values of the physiological parameter, wherein the second time-series represents a numerical prediction of the values of the physiological parameter ahead in time of the first time series. In other words, the machine learning model may be trained to predict a time-series of future values of a physiological parameter of a patient based on a time-series of past measured values of the physiological parameter. The prediction system may use the machine learning model to generate, using a time-series of measured values from the real-time or near real-time stream as input, a time-series of predicted values as output. This time-series of predicted values may represent a prediction of the value of the physiological parameter for a number of time instances in the future. Depending on the configuration of the machine learning model and the prediction system, the time-series may span a particular time period in the future, e.g., 40 seconds or 20 minutes or 1 hour, and may have a particular time resolution, e.g., one predicted value (sample) per second or one sample per 10 seconds, one sample per minute, one sample per five minutes, etc.

Such a time-series of predicted values may be of clinical relevance to medical personnel, particularly in situations where the state of the patient may be unstable, for example in an emergency situation. While a single prediction of a value of the physiological parameter in the future may also be of some clinically relevance, such a single prediction does not clearly convey the future trend nor does it show further changes in the physiological parameter in the future, which may also be of clinical relevance.

However, it may require sufficient time to perceive and understand a time-series of predicted values if they were displayed. Such time is not always available, for example in the aforementioned emergency situation or when medical personnel have to care for a large number of patients. In such situations, the primary interest of medical personnel may be whether the physiological parameter of the patient is still within a normal or acceptable physiological range, with the exact numerical value of the physiological parameter being of secondary importance. The above measures may incorporate this insight by, instead of directly outputting the time-series of predicted values, classifying the individual predicted values as being within the physiological range or outside of said range, thereby obtaining a time-series of classifications. Compared to the time-series of predicted values, this time-series of classifications may be easier to perceive and understand. For example, it may not be needed to mentally interpret numerical values, but rather, only a limited number of class labels may be interpreted, e.g., class labels indicating a predicted value falling outside or inside of the range. Such class labels may typically also be visualized in a clear manner, e.g., as distinct colors (e.g., green, or red) and may be more easily visualized by a graphical representation of a patient such as the one described by US 10702214B2.

While a machine learning model may also be directly trained to provide such a classification, the above measures apply the classification outside of the machine learning model. This may be advantageous since this way, the physiological range used for the classification may be easily changed and thereby adapted to local and regional differences, without requiring the machine learning model to be retrained. In particular, such physiological ranges may represent alarm thresholds which may be specific for health care providers and/or medical personnel. The machine learning model may thereby remain agnostic to the exact values to which these alarm thresholds are set.

Optionally, the time-series of predicted values spans a time period selected between seconds and tens of minutes, for example selected between 10 seconds and 1 hour. The time period may also be referred to as a prediction horizon. A prediction horizon between 10 seconds and 1 hour has been found to be clinically relevant for various types of physiological parameters.

Optionally, the time-series of predicted values has a time resolution selected between half a second and minutes, for example comprising predicted values for every second, every 10 seconds, every minute, or every five minutes. Such a time resolution may also be referred to as a sampling rate and may also be expressed in Hz.

Optionally, the prediction system is configured to access at least two machine learning models, wherein a first machine learning model is configured to make shorter term predictions and a second machine learning model is configured to make longer term predictions, wherein the processor subsystem is configured to combine outputs of the first machine learning model and the second machine learning model to obtain one time-series of classifications to be provided to the clinical decision support tool. Both a short prediction horizon and a long prediction horizon may be clinically relevant. It may be advantageous to provide separate machine learning models for the respective prediction horizons, for example to account for the fact that the training data for such prediction horizons may be different. A short prediction horizon may be for example be advantageous when predicting vital sign deviations for surgical patients undergoing anesthesia. For example, a hypotension prediction perioperatively becomes significant only when an adverse event is predicted with a very high probability seconds to a few minutes before the event. When such an adverse event is predicted, it is possible to prepare and quickly counteract hypotension when it arises. However, it is unlikely that an anesthesiologist would give a patient a vasopressor 20 minutes in advance when hypotension is forecasted in 20 minutes. In other words, an anesthesiologist may be reluctant to act for predictions far in the future, but may still be interested in having such predictions, e.g., to be able to identify a longer-term trend. Another advantage of having a machine learning model with a short prediction horizon is that such a model may be quicker to start outputting predictions as it may have a lower start-up latency. In clinical use, this means that such a model may need less time to start outputting predictions when a data stream from a new patient is arriving in real-time.

Optionally, the first machine learning model is configured to output a first time-series of predicted values which has a first time resolution selected between half a second and tens of seconds, and wherein the second machine learning model is configured to output a second time-series of predicted values which has a second time resolution selected between tens of seconds and minutes. Here, the term 'tens of seconds' may refer to any one of: 10, 20, 30, 40 and 50 seconds.

Optionally, the machine learning model is trained to predict the time-series of values of the physiological parameter using a further time series of values of a further physiological parameter as further input. The past measured values of a second physiological parameter may also be predictive for the future values of the first physiological parameter. Accordingly, the machine learning model may be trained to use the past measured values of both types of physiological parameters to provide a prediction of future values of the first physiological parameter. Advantageously, a more accurate prediction of the future values of the first physiological parameter is obtained.

Optionally, the physiological parameter is one of: temperature, heart rate, pulse rate, ST-segment deviation, invasive arterial blood pressure, central venous pressure, respiratory rate, tidal volume, end-tidal CO2 concentration, train of four ratio, bispectral index, oxygen saturation, and non-invasive arterial blood pressure.

Optionally, the processor subsystem of the training system is configured to select, in respective training iterations, non-overlapping instances of the first time series of values of the physiological parameter from the training data. By selecting time-series of measured values as input which do not overlap between the iterations of a training epoch, so-called data leakage may be avoided which may otherwise cause the performance of the machine learning model to be worse at prediction time. In other words, this way, statistical independence of the input samples may be ensured.

Optionally, the processor subsystem of the training system is configured to, in the training iteration, apply an outlier detection technique to the first time-series of values of the physiological parameter, and if the first time-series of values of the physiological parameter is classified as an outlier with respect to the training data, apply a higher weight to an output of the machine learning model in the training than when the first time-series of values of the physiological parameter is not classified as an outlier with respect to the training data. Many patterns in the development of physical parameters over time only occur seldom in real-life but may nevertheless be highly clinically relevant, e.g., as they may indicate a quick deterioration in medical condition or a particular medical problem. Due to the infrequency of their occurrence in real-life, they may also seldomly occur in the training data, causing the machine learning model to fail to accurately predict future values in such situations. To address this problem, seldomly occurring situations may be detected in the training data using an outlier detection technique, and if an outlier is detected, the weight assigned to the training error in the training may be increased so as to incentivize the optimization algorithm to more accurately predict the future values of the physiological parameter. This way, the prediction system provides more accurate predictions in seldomly occurring situations.

Optionally, the machine learning model is one of: recurrent neural network, such as a long short-term memory neural network, a logistic regression-based model, and a decision-tree based model.

It will be appreciated by those skilled in the art that two or more of the above-mentioned embodiments, implementations, and/or optional aspects of the invention may be combined in any way deemed useful.

Modifications and variations of any of the systems, any of the computer-implemented methods and/or any of the computer programs, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system configured access a real-time or near real-time data stream of measured values of a physiological parameter of a patient and to generate and provide a time-series of classifications to a clinical decision support tool;
Fig. 2 shows a system for training a machine learning model to predict future values of a physiological parameter of a patient based on past measured values of the physiological parameter;
Fig. 3A shows several measured physiological parameters displayed by a patient monitor;
Figs. 3B and 3C shows two time-series of the measured values of a physiological parameter being selected for the training of the machine learning model, wherein the first time-series of measured values is selected as input and the second time-series of measured values, which is ahead in time with respect to the first time-series, is selected as a training target;
Fig. 4A shows a time-series of predicted values of a physiological parameter, obtained by applying the machine learning model to a time-series of measured values of the physiological parameter;
Fig. 4B shows a comparison of the time-series of predicted values of the physiological parameter against a physiological range define by an upper bound and a lower bound;
Fig. 4C shows a time-series of classifications which are indicative of a respective predicted value falling inside, above or below the physiological range.
Fig. 5 shows a method of providing a prediction for a physiological parameter;
Fig. 6 shows a method of training a machine learning model; and
Fig. 7 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 10: physiological parameter
- 12: data stream of measured values of physiological parameter
- 20: time series of classifications
- 30: training data
- 40: data defining machine learning model
- 50: clinical decision support tool

- 100: prediction system for providing prediction for physiological parameter to clinical decision support tool
- 120: sensor interface
- 140: processor subsystem
- 160: memory
- 180: output interface

- 200: training system for training machine learning model
- 220: input interface
- 230: data storage
- 240: processor subsystem
- 260: memory

- 300: display area showing measured physiological parameters
- 310: time axis
- 312: value axis
- 320: data stream of measured values of physiological parameter
- 322: first time-series of measured values
- 324: second time-series of measured values
- 330: numerical visualization of current measured value
- 340: time series of predicted values
- 350: predicted value
- 360: physiological range
- 362: lower bound
- 364: upper bound
- 370: classification with respect to physiological range

- 400: method of providing prediction for physiological parameter
- 410: accessing data stream of measured values of physiological parameter
- 420: accessing data defining machine learning model
- 430: generating time series of predicted values
- 440: generating time series of classifications
- 450: providing classifications to clinical decision support tool

- 500: method of training machine learning model
- 510: accessing training data
- 520: iteratively training machine learning model
- 530: selecting first time-series of physiological parameter values
- 540: selecting second time-series of physiological parameter values
- 550: training machine learning model to predict second time-series

- 600: non-transitory computer-readable medium
- 610: data representing computer program

### DETAILED DESCRIPTION OF EMBODIMENTS

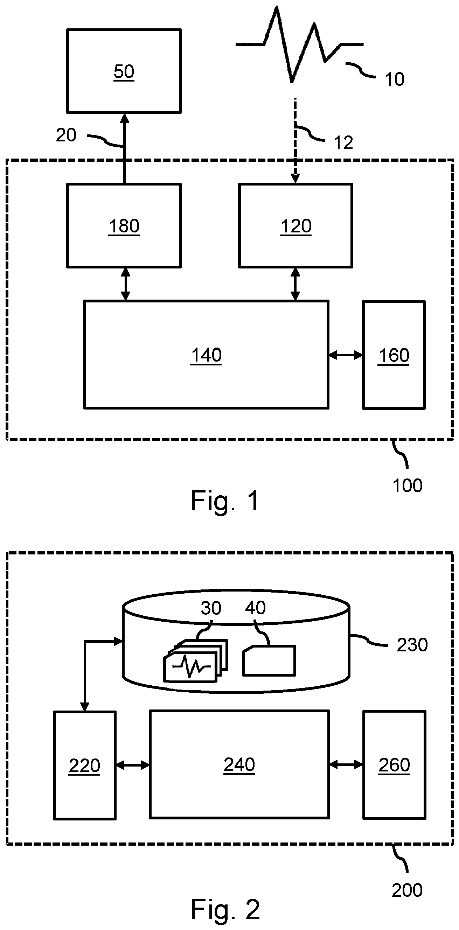

Fig. 1 shows a prediction system 100 for providing a prediction for a physiological parameter of a patient and for outputting the prediction to a clinical decision support tool. The prediction system 100 may comprise an input interface 120 configured to access a data stream 12 of measured values of a physiological parameter 10 of the patient. Such a data stream 12 may be received in real-time or near real-time and may for example be received from a patient monitor (not shown in Fig. 1) which is connected to one or more sensors to measure physiological signals of the patient, or directly from one or more of such sensors. Examples of sensors include, but are not limited to, a Swan Ganz pulmonary artery catheter (PAC), Philips PiCCO (which combines an arterial line and a central venous line), Edwards ClearSight (which uses finger cuffs), Edwards FloTrac (using an arterial line), echography, Philips ShellCuff (using a cuff around the upper arm), etc. The input interface 120 may for example be a network interface such as an ethernet interface or a bus-type interface such as an USB interface. In some examples, the prediction system 100 may be part of a patient monitor, in which case the input interface 120 may be an internal interface.

The prediction system 100 may further comprise a memory 160. The memory 160 may serve as short term data storage for the prediction system 100. For example, the data stream 12 received via the input interface 120 may be buffered, that is at least temporarily stored, in the memory 160. Another example is that instruction data may be stored in the memory 160 for execution by a processor subsystem of the prediction system 100. The memory 160 may for example be a volatile memory or a persistent memory, e.g., in form of a flash memory. Although not shown in Fig. 1, the prediction system 100 may also comprise a further data storage which may be used for longer term storage. Such a data storage may take any suitable form, such as a hard disk or a solid-state disk, or any array of such disks. It is noted that the further data storage may also be used for short term data storage, for example to buffer the data stream 12 received via the input interface 120.

The prediction system 100 may further comprise an output interface 180 to a clinical decision support tool 50. The clinical decision support tool 50 may for example provide a graphical representation of the patient to support medical personnel in taking clinical decisions. However, this is not a limitation, in that the clinical decision support tool 50 may also take any other form in which support for clinical decision making is provided. The clinical decision support tool 50 may be implemented by software and/or hardware. For example, the clinical decision support tool 50 may be implemented by a patient monitor. In such a case, the input interface 120 and the output interface 180 may be part of a same input-output interface to the patient monitor. Another example is that the clinical decision support tool 50 may be implemented as software running on a PC, laptop, workstation, or server. The output interface 180 may be of any suitable type, for example a network interface such as an ethernet interface or a bus-type interface such as an USB interface. In some examples, the prediction system 100 may be part of a patient monitor, in which case the output interface 180 may be an internal interface of the prediction system 100.

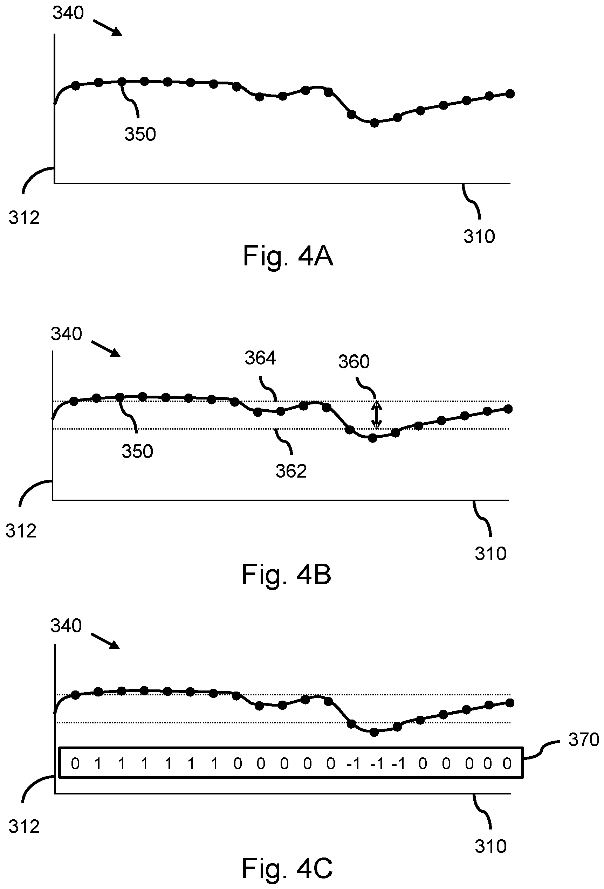

The prediction system 100 is further shown to comprise a processor subsystem 140 configured to internally communicate with the input interface 120, with the memory 160 and with the output interface 180. As also described with reference to Fig. 4A-4C and elsewhere, the processor subsystem 140 may be configured, for example by instruction data stored in the memory 160, to use a machine learning model to generate, using a time-series of measured values from the real-time or near real-time stream 12 as input, a time-series of predicted values as output, determine for the time-series of predicted values whether predicted values fall within a physiological range associated with the physiological parameter, thereby obtaining a time-series of classifications 20, wherein a respective classification is indicative of whether a predicted value falls inside or outside of the physiological range, and provide the time-series of classifications to the clinical decision support tool 50. These and other operations of the prediction system 100, and various optional aspects thereof, will be explained in more detail elsewhere in this specification.

**Fig.** 2 shows a training system 200 for training a machine learning model to predict future values of a physiological parameter of a patient based on past measured values of the physiological parameter. The machine learning model trained by the training system 200 may be used by the prediction system 100 to generate the time-series of predicted values. The training system 200 may comprise a memory 260. The memory 260 may serve as short term data storage for the training system 200. For example, instruction data may be stored in the memory 260 for execution by a processor subsystem of the training system 200. The memory 260 may for example be a volatile memory or a persistent memory, e.g., in form of a flash memory. The training system 100 may further comprise an input interface 220 configured to access training data 30 in form of recordings of real-time or near real-time data streams of measured values of a physiological parameter of one or more patients. In the example of Fig. 2, the input interface 220 is shown to be a data storage interface to an internal data storage 230 which comprises the training data 30. The data storage 230 may for example be a hard disk or a solid-state disk, or any array of such disks, and the data storage interface 220 may be a corresponding type of interface. Another example is that the input interface 220 may be a network interface, such as an ethernet interface, to a network-accessible data storage which comprises the training data 230.

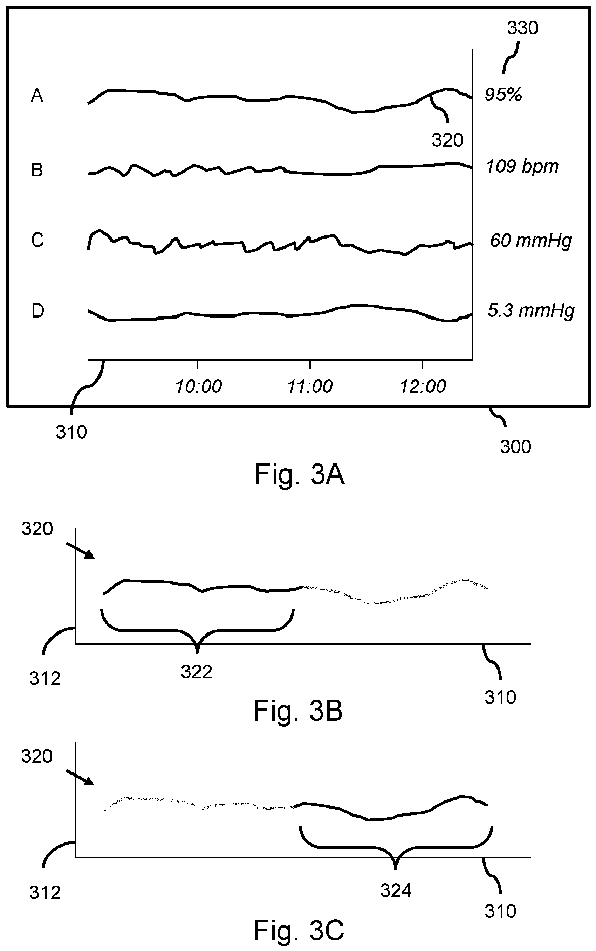

The training system 200 is further shown to comprise a processor subsystem 240 configured to internally communicate with the input interface 220 and with the memory 260. As also described with reference to Fig. 3A-3C and elsewhere, the processor subsystem 240 may be configured, for example by instruction data stored in the memory 260, to iteratively training a machine learning model on the training data to obtain a machine learning model by, in a training iteration, selecting a first time-series of values of the physiological parameter from the training data, selecting a second time-series of values of the physiological parameter from the training data, wherein the second time series is part of a same data stream as the first time-series of values of the physiological parameter and is ahead in time of the first time series, and training the machine learning model to predict the second time-series of values of the physiological parameter from the first time-series of values of the physiological parameter as input. The trained machine learning model may for example be stored as data 40 in the data storage 230 or output in any other manner.

In general, each of the prediction system 100 and the training system 200 may be embodied as, or in, a single device or apparatus. The device or apparatus may be a general-purpose device or apparatus, such as a workstation or a computer, but may also be application-specific, such as a patient monitor. The device or apparatus may comprise one or more microprocessors which may represent the processor subsystem, and which may execute appropriate software. The software may have been downloaded and/or stored in a corresponding memory, e.g., a volatile memory such as RAM or a non-volatile memory such as Flash. Alternatively, the functional units of the system, e.g., the input interface, the user interface subsystem, and the processor subsystem, may be implemented in the device or apparatus in the form of programmable logic, e.g., as a Field-Programmable Gate Array (FPGA). In general, each functional unit of the prediction system 100 and of the training system 200 may be implemented in the form of a circuit. It is noted that each of the prediction system 100 and the training system 200 may also be implemented in a distributed manner, e.g., involving different devices or apparatuses. For example, the distribution may be in accordance with a client-server model, e.g., using a server and workstation.

Fig. 3A shows an example of measurements of physiological parameters 320 which may be displayed by a patient monitor in a display area 300 of a display. In this example, the measurements of the physiological parameters 320 are displayed along a horizontal axis 310 along which time is set out and which axis may also be referred to as time axis, and a vertical axis along which the measured values of the physiological parameters are set out, being in this example from top to bottom the blood oxygen saturation, mean arterial pressure, the heart rate, and the central venous pressure. Typically, during operation of the patient monitor, the current or most recently measured values of the physiological parameters are plotted at the right-hand side of the display area 300 while past measured values are over time moved toward the left-hand side. Typically, the current or most recent measured values of the physiological parameters are numerically shown 330 at the right-hand side of the display area 300, showing that the current or most recent measured values are respectively "95%", "60 mmHg", "109 bpm", and "5.3 mmHg".

As elucidated elsewhere in this specification, it may be desirable to be able to provide a prediction for a physiological parameter of a patient for use by a clinical decision support tool. In some examples, the clinical decision support may also be implemented by a patient monitor, for example the patient monitor showing a graphical representation of the patient which visually conveys a current state of the patient, while in other examples, the clinical decision support tool may be implemented outside of a patient monitor.

To obtain a prediction for the physiological parameter, a machine learning model may be trained, for example by the training system 200 of Fig. 2. As input to the training, training data may be used, which training data may comprise recordings of real-time or near real-time the data streams of measured values of a physiological parameter of one or more patients. For example, one or more of the data streams visualized by the patient monitor as shown in Fig. 3A may be recorded and used for training purposes. In some examples, the data streams of several patients may be used. In some examples, the training data may comprise data from several care centers, for example from several hospitals. The training may comprise several iterations, with an iteration involving training the machine learning model on a particular part of the training data. In some examples, the training may pass through the training data several times, e.g., in respective epochs.

Figs. 3B and 3C illustrates the data selection for an iteration of the training, with Fig. 3B showing a first time-series 322 of values of the physiological parameter having been selected from the data stream 320. This first time-series may represent a subset, e.g., a segment, selected from the data stream, being in this example a data stream of measured blood oxygen values. For example, the first time-series may be comprised of tens of samples, with each sample representing a measured value. The samples may be selected as consecutive samples from the data stream, but may in some examples also be selected by subsampling the data stream. For example, every second, third, fourth, fifth, 10^{th}, etc. sample from the data stream may be selected. The first time-series 322 may serve as input to the machine learning model during the training iteration. In addition, a second time-series may be selected to serve as prediction target in the training iteration. As shown in Fig. 3C, the second time-series 324 may be selected as a set of samples which are ahead in time of the first time-series 322. For example, the second time-series 324 may immediately follow the first time-series 322, or in some examples may be minorly spaced apart in time.

Having selected the first time-series 322 and the second time-series 324 of measured values from the data stream 320, the machine learning model may be trained with the first time-series 322 being used as input to the machine learning model and the second time-series 324 as prediction target. Accordingly, the machine learning model may be trained to predict the second time-series 324 using the first time-series 322 as input. Thereby, the machine learning model may be trained to predict a time-series of future values of the physiological parameter, being in this example the blood oxygen saturation, based on a time-series of current and/or past measured values. The training process may continue by selecting, in a next iteration of the training, another time-series of measured values as input and yet another time-series of measured values as prediction target, which process may repeat itself for a number of iterations. In some embodiments, the time-series which are selected as input during the training may be selected not to overlap in time, meaning that each iteration in an epoch of the training uses a different part of the training data as input.

With continued reference to the selection of the first time-series and the second time-series, it is noted that the length of the first time-series and the second time-series, e.g., in number of samples, may be the same, for example 20 samples each. However, this is not a limitation, in that the lengths may also be selected differently. For example, the length of the second time-series may be selected to be equal to or shorter than the length of the first time-series. For example, 20 samples may be used as input to the machine learning model to train the model to predict 10 samples ahead in the future.

In some specific examples, the machine learning model may be trained for one of the following prediction tasks (which may also be considered regression tasks by the prediction involving the prediction of continuous values): prediction of 10, 20, and 40 seconds ahead (using 1-sec data, e.g., a sample rate of 1 Hz) and 5, 10 and 20 minutes ahead (using 1-min data, e.g., a sample rate of 1/60 Hz). Accordingly, the machine learning model may, when trained to predict 20 minutes ahead, receive 20 samples covering 20 minutes of the data stream as input and may predict, from this input, the values of the physiological parameter as 20 samples covering the next 20 minutes. In other examples, two or more machine learning models may be trained, each having a different prediction horizon, referring to the time period which is predicted ahead, and/or having a different time resolution, referring to the time by which individual samples are spaced apart in time. For example, a first machine learning model may be trained to predict 40 seconds ahead with a sampling rate of 1 Hz, meaning that the first machine learning model may provide an array of 40 samples as output, while a second machine learning model may be trained to predict 20 minutes ahead with a sampling rate of 1/60 Hz (e.g., 1 minute), meaning that the second machine learning model may provide an array of 20 samples as output. The outputs of both machine learning models may be combined as described elsewhere in this specification.

In some embodiments, an outlier detection technique may be applied to the input data during the training. Such an outlier detection technique may be based on a statistical analysis of the training data, and may indicate whether a first time-series to be used as input in a training iteration is considered to be an outlier with respect to the training data, or with respect to a part of the training data. Such outlier detection is known per se, and any suitable outlier detection technique may be used for this purpose. If the first time-series of values of the physiological parameter is classified as an outlier by the outlier detection technique, a higher weight may be applied to an output of the machine learning model in the training compared to the case that the first time-series would not be classified as an outlier. Accordingly, the training error in this training iteration may be given a higher weight than in a training iteration in which the first time-series is not an outlier.

The machine learning model may take any suitable form. For example, the machine learning model may be a neural network, such as a recurrent neural network. In a specific example, the neural network may be a deep recurrent neural network which uses a long-short-term-memory layer which processes the input data, e.g., the samples of the first time-series, and computes an inner representation of the input data. The neural network may further comprise a dense layer as output layer to compute the prediction, e.g., the samples of the second time-series. The size of the dense layer may be selected in accordance with the prediction horizon, for example equal the number of samples to be predicted. For example, for a prediction of 20 minutes ahead with a sampling rate of 1 sample per minute, a dense layer of size 20 may be used. In other examples, the machine learning model may be a logistic regression-based model or a decision-tree based model.

Having trained the machine learning model, the trained machine learning model may be made accessible to the prediction system as described elsewhere in this specification. The prediction system may use the trained machine learning model to provide a prediction for a physiological parameter of a patient and output the prediction to a clinical decision support tool. This may involve applying the trained machine learning model to a time-series of measured values from a real-time or near real-time data stream as input, for example on a continuous or periodical basis. For example, the data stream may be a stream of measured blood oxygen saturation values as shown in Figs. 3A-3C. The time-series of measured values may be selected to comprise a current or most recent measured value and a number of preceding measured values. In general, the time period and/or the time resolution of the series of measured values may be selected to, but does not need to, correspond to the time period and/or the time resolution of the respective first time-series which are used as input during the training of the machine learning model.

**Fig. 4A** shows a time-series of predicted values 340 of a physiological parameter, obtained by applying the machine learning model to the aforementioned time-series of measured values of the physiological parameter. The time-series 340 is shown to be comprised of 20 individual samples 350, which may for example cover a shorter time period of 20 seconds or a longer time period of 20 minutes or 1 hour. It is noted that although Figs. 4A-4C show the predicted values 340 as a continuous graph, the continuous graph is merely generated for illustrative purposes by interpolation of the individual samples 350. This also applies to Figs. 3A-3C where likewise continuous graphs are shown for illustrative purposes.

Instead of directly outputting the time-series of predicted values 340 to the clinical decision support tool, the prediction system may first determine for the time-series of predicted values whether the predicted values fall within a physiological range associated with the physiological parameter, thereby obtaining a time-series of classifications, wherein a respective classification is indicative of whether a predicted value falls inside or outside of the physiological range. Such a physiological range may for example correspond to a normal or acceptable physiological range and may in some examples be user-definable, for example as user-definable parameters which are accessible via a graphical user interface.

**Fig. 4B** shows this comparison of the time-series of predicted values of the physiological parameter against a physiological range 360 defined by an upper bound 362 and a lower bound 364. It can be seen that some of the samples 350 exceed the upper bound 362, that other samples fall below the lower bound 364, and that yet other samples remain within the physiological range 360. **Fig. 4C** shows a time-series of classifications 370 which may be generated by comparison with the physiological bound and which are indicative of a respective predicted value falling inside, above or below the physiological range. In this example, the classification has resulted in numerical class labels having been assigned a value in a ternary classification system, e.g., '0' for falling within the physiological range, '1' for exceeding the upper limit, and `-1' for falling below the lower limit. These classifications together may form a time-series of classifications 370 which may be output by the prediction system to the clinical decision support tool. It will be appreciated that any other suitable type of classification may be used instead, for example using other numerical labels, using non-numerical labels, using more or fewer classes, etc. An example of the use of fewer classes may be the assignment of a class label of '0' for falling within the physiological range and '1' for falling outside of the physiological range, or vice versa. The time-series of classifications 370 may be output to the clinical decision support tool directly, e.g., as an array of class labels, or after conversion or encoding. An example of conversion is that an array of binary or ternary values may be converted into a decimal number.

It is noted that the prediction system and method may make use of two or more machine learning models. For example, a first machine learning model may be trained to make shorter term predictions and a second machine learning model may be trained to make longer term predictions. In a specific example, the first machine learning model may have a prediction horizon up to 1 minute and the second machine learning model may have a prediction horizon above 1 minute. In a yet more specific example, the first machine learning model may have a prediction horizon of 40 seconds and a time resolution of 1 sample per second and the second machine learning model may have a prediction horizon of 20 minutes and a time resolution of 1 sample per minute. In another specific example, the second machine learning model may have a prediction horizon of 100 minutes and a time resolution of 1 sample per 5 minutes. The predictions of both models may be combined into a single time-series of predicted values, e.g., by concatenation, and then classified as described above with reference to Figs. 4A-4C. It is noted that both model outputs may also be individually classified, and their resulting time-series of classifications may be combined, e.g., by concatenation, into a single time-series of classifications to be provided to the clinical decision support tool. Alternatively, the prediction system and method may provide the outputs of both models separately to the clinical decision support tool.

Several machine learning models were trained using measurement data obtained from a tertiary care center in Zurich, Switzerland, which uses a patient data management system that captures hemodynamic and ventilatory parameters from patient monitors and the anesthesia machines. In the training, 80% of the measurement data was used as training data while 20% of the measurement data was used as test data for validating the performance of the machine learning models. The machine learning models were trained on the training data using data streams with a time resolution of 1 Hz (e.g., 1 sample per second) and a prediction horizon of 10, 20 and 40 seconds, and using data streams with a time resolution of 1/60 Hz (e.g., 1 sample per minute) and a prediction horizon of 5, 10 and 20 minutes. The validation involved, amongst other aspects, evaluating the balance accuracy of the time-series of classification generated by the prediction system and method. The balance accuracy corresponds to an average between sensitivity and specificity values (e.g., (*sensitivity* + *specificity*)/2) and, hence, serves as a single performance measure. Table 1 below shows the balance accuracy for the classifications obtained for machine learning models trained for different physiological parameters and for different prediction horizons and when applying the machine learning models to the validation data. It can be seen that most balance accuracy values are 0.85 or above, indicating (very) good performance, and that nearly all values are 0.71 or above, indicating at least acceptable performance. The table therefore demonstrates the good performance of the prediction system and method as described in this specification.

**Table 1. Balanced accuracy for different physiological parameters and different prediction horizons for the test data.**

| **Variable name** | **Prediction Horizon** | | | | | | | | | | | |
|---|---|---|---|---|---|---|---|---|---|---|---|---|
| | **10 see** | | **20 see** | | **40 see** | | **5 min** | | **10 min** | | **20 min** | |
| | **Low** | **High** | **Low** | **High** | **Low** | **High** | **Low** | **High** | **Low** | **High** | **Low** | **High** |
| Temperature (Temp) | 0.984 | 0.991 | 0.990 | 0.992 | 0.965 | 0.988 | 0.967 | 0.961 | 0.954 | 0.952 | 0.916 | 0.940 |
| Heart rate (HR) | 0.965 | 0.954 | 0.955 | 0.950 | 0.952 | 0.937 | 0.921 | 0.890 | 0.904 | 0.875 | 0.874 | 0.853 |
| Pulse (PR) | 0.742 | 0.925 | 0.717 | 0.924 | 0.679 | 0.920 | 0.819 | 0.896 | 0.807 | 0.875 | 0.786 | 0.844 |
| ST-segment deviation | 0.929 | 0.940 | 0.908 | 0.902 | 0.901 | 0.920 | 0.879 | 0.912 | 0.875 | 0.906 | 0.847 | 0.879 |
| Invasive arterial blood pressure (IABP) | 0.942 | 0.940 | 0.975 | 0.926 | 0.912 | 0.912 | 0.753 | 0.746 | 0.727 | 0.720 | 0.715 | 0.695 |
| Central venous pressure (CVP) | 0.924 | 0.940 | 0.936 | 0.938 | 0.913 | 0.930 | 0.864 | 0.887 | 0.851 | 0.870 | 0.813 | 0.838 |
| Respiratory rate (RR) | 0.825 | 0.885 | 0.876 | 0.851 | 0.788 | 0.781 | 0.920 | 0.770 | 0.907 | 0.762 | 0.890 | 0.734 |
| Tidal volume (TV) | 0.900 | 0.895 | 0.883 | 0.890 | 0.868 | 0.852 | 0.850 | 0.877 | 0.843 | 0.862 | 0.829 | 0.831 |
| End tidal CO2 concentration (etCO2) | 0.949 | 0.889 | 0.936 | 0.875 | 0.938 | 0.825 | 0.881 | 0.824 | 0.857 | 0.767 | 0.821 | 0.731 |
| Train of four ratio (TOF%) | 0.996 | 0.994 | 0.994 | 0.998 | 0.993 | 0.997 | 0.886 | 0.894 | 0.853 | 0.858 | 0.810 | 0.849 |
| Bispectral Index (BIS) | 0.931 | 0.927 | 0.911 | 0.907 | 0.913 | 0.899 | 0.838 | 0.833 | 0.810 | 0.806 | 0.774 | 0.772 |
| Oxygen saturation (SpO2) | 0.953 | 0.957 | 0.948 | 0.950 | 0.939 | 0.940 | 0.868 | 0.869 | 0.838 | 0.840 | 0.809 | 0.810 |
| Non-invasive arterial blood pressure (NIABP) | No data | No data | No data | No data | No data | No data | 0.685 | 0.668 | 0.650 | 0.618 | 0.607 | 0.585 |

The prediction system and method which use these machine learning models have been additionally validated using the elCU Collaborative Research Database (retrieved at https://physionet.org/content/eicu-crd/2.0/) and MIMIC-III Waveform Database (retrieved at https://physionet.org/content/mimic3wdb/1.0/). The validation used the vital sign measurements Temp, HR, ST-segment, IABP, CVP, RR, etCO2, and SpO2 stored as 1 sample per 5 minutes resolutions. In the validation, the machine learning model is used to predict the vital sign measurements up to 20 * 5 minutes = 100 minutes, or more than 1 hour, into the future, in addition to 25 and 50 minutes into the future. The MIMIC-III Waveform Database contained 67,830 record sets of multiple physiologic signals ("waveforms") and time series of vital signs ("numerics") for approximately 30,000 ICU patients collected from bedside patient monitors in adult and neonatal intensive care units (ICUs) of Beth Israel Deaconess Medical Center (Boston, MA). The validation used the "numerics" part of the database and used the vital sign recordings of SpO2, HR, RR, IABP, and PR available in 1 sample per minute time resolutions and SpO2, HR, RR, and PR available in 1 sample per second time resolutions. Table 2a shows the validation results for 10 sec, 20 sec, 40 sec, 5 min, 10 min, and 20 min prediction horizons for the data from MIMIC-III database and Table 2b for 25 min, 50 min, and 100 min for the elCU data.

**Table 2A. Balanced accuracy for different physiological parameters and different prediction horizons for the data from the MIMIC-III database.**

| **Variable name** | **Prediction horizon** | | | | | | | | | | | |
|---|---|---|---|---|---|---|---|---|---|---|---|---|
| | **10 see** | | **20 see** | | **40 see** | | **5 min** | | **10 min** | | **20 min** | |
| | Low | High | Low | High | Low | High | Low | High | Low | High | Low | High |
| Temperature (Temp) | No data | No data | No data | No data | No data | No data | No data | No data | No data | No data | No data | No data |
| Heart rate (HR) | 0.95 6 | 0.98 2 | 0.95 7 | 0.97 5 | 0.95 1 | 0.97 2 | 0.97 1 | 0.95 2 | 0.94 6 | 0.91 8 | 3.937 | 0.903 |
| Pulse rate (PR) | 0.93 3 | 0.98 4 | 0.89 9 | 0.97 9 | 0.87 2 | 0.97 2 | 0.94 0 | 0.94 7 | 0.93 8 | 0.92 9 | 3.934 | 0.924 |
| ST-segment deviation (ST-segment) | No data | No data | No data | No data | No data | No data | No data | No data | No data | No data | No data | No data |
| Invasive arterial blood pressure (IABP) | No data | No data | No data | No data | No data | No data | 0.86 9 | 0.90 5 | 0.83 7 | 0.88 7 | 3.788 | 0.865 |
| Central venous pressure (CVP) | No data | No data | No data | No data | No data | No data | No data | No data | No data | No data | No data | No data |
| Respiratory rate (RR) | 0.84 1 | 0.95 1 | 0.86 0 | 0.92 3 | 0.78 7 | 0.88 5 | 0.61 1 | 0.84 2 | 0.57 5 | 0.82 5 | 3.510 | 0.806 |
| Tidal volume (TV) | No data | No data | No data | No data | No data | No data | No data | No data | No data | No data | No data | No data |
| End-tidal CO2 concentratio n (etCO2) | No data | No data | No data | No data | No data | No data | No data | No data | No data | No data | No data | No data |
| Train of four ratio (TOF%) | No data | No data | No data | No data | No data | No data | No data | No data | No data | No data | No data | No data |
| Bispectral Index (BIS) | No data | No data | No data | No data | No data | No data | No data | No data | No data | No data | No data | No data |
| Oxygen saturation (SpO2) | 0.94 1 | 0.93 6 | 0.91 4 | 0.90 4 | 0.87 5 | 0.86 1 | 0.86 2 | 0.85 2 | 0.82 6 | 0.81 5 | 3.771 | 0.761 |
| Non-invasive arterial blood pressure (NIABP) | No data | No data | No data | No data | No data | No data | No data | No data | No data | No data | No data | No data |

**Table 2B. Balanced accuracy for different physiological parameters and different prediction horizons for the data from the eICU database.**

| **Variable name** | **Prediction horizon** | | | | | |
|---|---|---|---|---|---|---|
| | **25 min** | | **50 min** | | **100 min** | |
| | Low | High | Low | High | Low | High |
| Temperature (Temp) | 0.931 | 0.940 | 0.901 | 0.922 | 0.888 | 0.967 |
| Heart rate (HR) | 0.985 | 0.973 | 0.980 | 0.961 | 0.972 | 0.945 |
| Pulse rate (PR) | No data | No data | No data | No data | No data | No data |
| ST-segment deviation (ST-segment) | 0.923 | 0.915 | 0.900 | 0.904 | 0.882 | 0.892 |
| Invasive arterial blood pressure (IABP) | 0.842 | 0.866 | 0.825 | 0.871 | 0.819 | 0.834 |
| Central venous pressure (CVP) | 0.814 | 0.878 | 0.743 | 0.840 | 0.674 | 0.797 |
| Respiratory rate (RR) | 0.715 | 0.846 | 0.682 | 0.820 | 0.642 | 0.802 |
| Tidal volume (TV) | No data | No data | No data | No data | No data | No data |
| End-tidal CO2 concentration (etCO2) | 0.996 | 0.993 | 0.996 | 0.993 | 0.995 | 0.988 |
| Train of four ratio (TOF%) | No data | No data | No data | No data | No data | No data |
| Bispectral Index (BIS) | No data | No data | No data | No data | No data | No data |
| Oxygen saturation (SpO2) | 0.782 | 0.821 | 0.706 | 0.749 | 0.627 | 0.661 |
| Non-invasive arterial blood pressure (NIABP) | No data | No data | No data | No data | No data | No data |

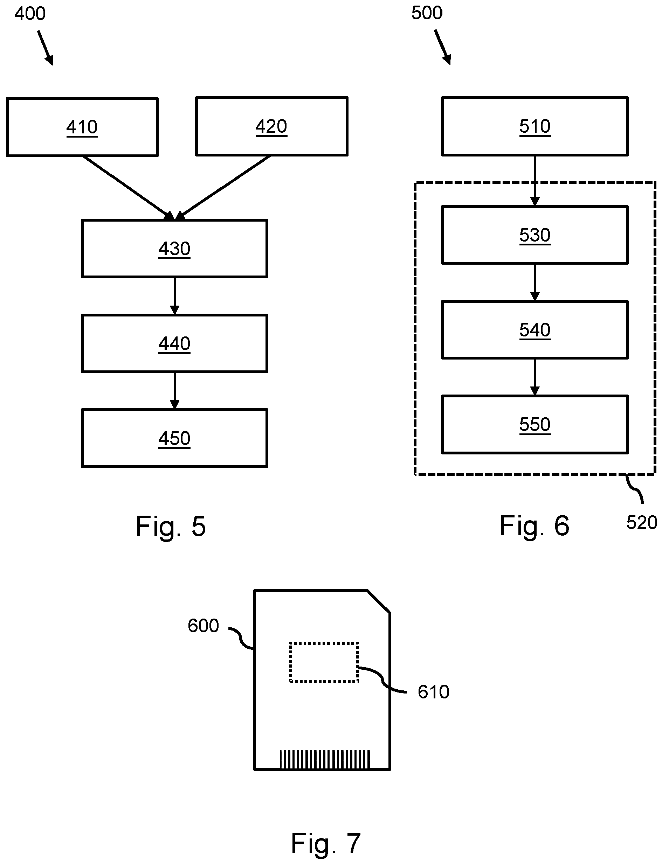

**Fig. 5** shows a block-diagram of computer-implemented method 400 for providing a prediction for a physiological parameter. The method 400 may correspond to an operation of the prediction system 100 of Fig. 1. However, this is not a limitation, in that the computer-implemented method 400 may also be performed using another system, apparatus or device. The method 400 is shown to comprise, in a step titled "ACCESSING DATA STREAM OF MEASURED VALUES OF PHYSIOLOGICAL PARAMETER", accessing 410 a real-time or near real-time data stream of measured values of a physiological parameter of the patient, in a step titled "ACCESSING DATA DEFINING MACHINE LEARNING MODEL", accessing 420 data defining machine learning model as defined elsewhere in this specification, in a step titled "GENERATING TIME SERIES OF PREDICTED VALUES", using the machine learning model to generate 430, using a time-series of measured values from the real-time or near real-time stream as input, a time-series of predicted values as output, in a step titled "GENERATING TIME SERIES OF CLASSIFICATIONS", determining for the time-series of predicted values whether predicted values fall within a physiological range associated with the physiological parameter, thereby obtaining 440 a time-series of classifications, wherein a respective classification is indicative of whether a predicted value falls inside or outside of the physiological range, and in a step titled "PROVIDING CLASSIFICATIONS TO CLINICAL DECISION SUPPORT TOOL", providing 450 the time-series of classifications to the clinical decision support tool.

**Fig. 6** shows a block-diagram of computer-implemented method 500 for training a machine learning model. The method 500 may correspond to an operation of the training system 200 of Fig. 2. However, this is not a limitation, in that the computer-implemented method 500 may also be performed using another system, apparatus or device. The method 500 is shown to comprise, in a step titled "ACCESSING TRAINING DATA", accessing 510 training data in form of recordings of real-time or near real-time data streams of measured values of a physiological parameter of one or more patients, in a step titled "ITERATIVELY TRAINING MACHINE LEARNING MODEL", iteratively training 520 the machine learning model on the training data to obtain a machine learning model by, in a training iteration and in a sub-step titled "SELECTING FIRST TIME-SERIES OF PHYSIOLOGICAL PARAMETER VALUES", selecting 530 a first time-series of values of the physiological parameter from the training data, in a sub-step titled "SELECTING SECOND TIME-SERIES OF PHYSIOLOGICAL PARAMETER VALUES", selecting 540 a second time-series of values of the physiological parameter from the training data, wherein the second time series is part of a same data stream as the first time-series of values of the physiological parameter and is ahead in time of the first time series, and in a sub-step titled "TRAINING MACHINE LEARNING MODEL TO PREDICT SECOND TIME-SERIES", training 550 the machine learning model to predict the second time-series of values of the physiological parameter from the first time-series of values of the physiological parameter as input.

It will be appreciated that in general, steps of the computer-implemented method 400 of Fig. 5 and the computer-implemented 500 of Fig. 6 may be performed in any suitable order, e.g., consecutively, simultaneously, or a combination thereof, subject to, where applicable, a particular order being necessitated, e.g., by input/output relations. Steps may also be performed as part of other steps, e.g., as sub-steps.

The method may be implemented on a computer as a computer implemented method, as dedicated hardware, or as a combination of both. As also illustrated in **Fig. 7****,** instructions for the computer, e.g., executable code, may be stored on a computer readable medium 600, e.g., in the form of a series 610 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 7 shows a memory device 600.

Examples, embodiments or optional features, whether indicated as nonlimiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A prediction system (100) for providing a prediction for a physiological parameter of a patient and for outputting the prediction to a clinical decision support tool (50), comprising:
- an input interface (120) configured to access a real-time or near real-time data stream of measured values of a physiological parameter of the patient;
- a data storage (160) comprising data defining machine learning model, wherein the machine learning model is configured to predict, using a first time-series of values of the physiological parameter as input, a second time-series of values of the physiological parameter, wherein the second time-series represents a numerical prediction of the values of the physiological parameter ahead in time of the first time series;
- an output interface (180) to the clinical decision support tool;
- a processor subsystem (140) configured to:
- use the machine learning model to generate, using a time-series of measured values from the real-time or near real-time stream as input, a time-series of predicted values (340) as output;
- determine for the time-series of predicted values whether predicted values fall within a physiological range (360) associated with the physiological parameter, thereby obtaining a time-series of classifications (370), wherein a respective classification is indicative of whether a predicted value falls inside or outside of the physiological range; and
- provide the time-series of classifications to the clinical decision support tool.

2. The prediction system (100) according to claim 1, wherein the time-series of predicted values (340) spans a time period selected between seconds and tens of minutes, for example selected between 10 seconds and 1 hour.

3. The prediction system (100) according to claim 1 or 2, wherein the time-series of predicted values (340) has a time resolution selected between half a second and minutes, for example comprising predicted values for every second, every 10 seconds, every minute, or every five minutes.

4. The prediction system (100) according to any one of claims 1 to 3, wherein the prediction system is configured to access at least two machine learning models, wherein a first machine learning model is configured to make shorter term predictions and a second machine learning model is configured to make longer term predictions, wherein the processor subsystem is configured to combine outputs of the first machine learning model and the second machine learning model to obtain one time-series of classifications to be provided to the clinical decision support tool.

5. The prediction system (100) according to claim 4, wherein the first machine learning model is configured to output a first time-series of predicted values which has a first time resolution selected between half a second and tens of seconds, and wherein the second machine learning model is configured to output a second time-series of predicted values which has a second time resolution selected between tens of seconds and minutes.

6. The prediction system (100) according to any one of claims 1 to 5, wherein the machine learning model is trained to predict the time-series of values of the physiological parameter using a further time series of values of a further physiological parameter as further input.

7. The prediction system (100) according to any one of claims 1 to 6, wherein the physiological parameter is one of: temperature, heart rate, pulse rate, ST-segment deviation, invasive arterial blood pressure, central venous pressure, respiratory rate, tidal volume, end-tidal CO2 concentration, train of four ratio, bispectral index, oxygen saturation, and non-invasive arterial blood pressure.

8. A patient monitor comprising the prediction system (100) according to any one of claims 1 to 7.

9. A training system (200) for training a machine learning model to predict future values of a physiological parameter of a patient based on past measured values of the physiological parameter, comprising:
- an input interface (220) configured to access training data (30) in form of recordings of real-time or near real-time data streams of measured values of a physiological parameter of one or more patients;
- a processor subsystem (240) configured to iteratively train the machine learning model on the training data to obtain a machine learning model by, in a training iteration:
- selecting a first time-series of values (322) of the physiological parameter from the training data;
- selecting a second time-series of values (324) of the physiological parameter from the training data, wherein the second time series is part of a same data stream as the first time-series of values of the physiological parameter and is ahead in time of the first time series; and
- training the machine learning model to predict the second time-series of values of the physiological parameter from the first time-series of values of the physiological parameter as input.

10. The training system (200) according to claim 9, wherein the processor subsystem (240) is configured to select, in respective training iterations, non-overlapping instances of the first time series of values of the physiological parameter from the training data.

11. The training system (200) according to claim 9 or 10, wherein the processor subsystem (240) is configured to, in the training iteration, apply an outlier detection technique to the first time-series of values of the physiological parameter, and if the first time-series of values of the physiological parameter is classified as an outlier with respect to the training data, apply a higher weight to an output of the machine learning model in the training than when the first time-series of values of the physiological parameter is not classified as an outlier with respect to the training data.

12. The training system (200) according to any one of claims 9 to 11, wherein the machine learning model is one of: recurrent neural network, such as a long short-term memory neural network, a logistic regression-based model, and a decision-tree based model.

13. A computer-implemented method (400) for providing a prediction for a physiological parameter of a patient and for providing the prediction to a clinical decision support tool, comprising:
- accessing (410) a real-time or near real-time data stream of measured values of a physiological parameter of the patient;
- accessing (420) data defining machine learning model, wherein the machine learning model is configured to predict, using a first time-series of values of the physiological parameter as input, a second time-series of values of the physiological parameter, wherein the second time-series represents a numerical prediction of the values of the physiological parameter ahead in time of the first time series;
- using the machine learning model to generate (430), using a time-series of measured values from the real-time or near real-time stream as input, a time-series of predicted values as output;
- determining for the time-series of predicted values whether predicted values fall within a physiological range associated with the physiological parameter, thereby obtaining (440) a time-series of classifications, wherein a respective classification is indicative of whether a predicted value falls inside or outside of the physiological range; and
- providing (450) the time-series of classifications to the clinical decision support tool.

14. A computer-implemented method (500) for training a machine learning model to predict future values of a physiological parameter of a patient based on past measured values of the physiological parameter, comprising:
- accessing (510) training data in form of recordings of real-time or near real-time data streams of measured values of a physiological parameter of one or more patients;
- iteratively training (520) the machine learning model on the training data to obtain a machine learning model by, in a training iteration:
- selecting (530) a first time-series of values of the physiological parameter from the training data;
- selecting (540) a second time-series of values of the physiological parameter from the training data, wherein the second time series is part of a same data stream as the first time-series of values of the physiological parameter and is ahead in time of the first time series; and
- training (550) the machine learning model to predict the second time-series of values of the physiological parameter from the first time-series of values of the physiological parameter as input.

15. A transitory or non-transitory computer-readable medium (600) comprising data (610) representing a computer program, the computer program comprising instructions for causing a processor system to perform the method according to claim 13 or 14.
